(19) Europäisches Patentamt
European Patent Office
Office européen des brevets

(11) Veröffentlichungsnummer: **0 040 344 B1**

(12) # EUROPÄISCHE PATENTSCHRIFT

(45) Veröffentlichungstag der Patentschrift: **05.12.84**

(51) Int. Cl.³: **C 07 D 213/89**, A 01 N 43/40

(21) Anmeldenummer: **81103315.8**

(22) Anmeldetag: **02.05.81**

(54) **4-Nitro-2-trichlormethylbenzolsulfensäurederivat, Verfahren zu seiner Herstellung, und dieses enthaltende Fungizide.**

(30) Priorität: **16.05.80 DE 3018716**

(43) Veröffentlichungstag der Anmeldung: **25.11.81 Patentblatt 81/47**

(45) Bekanntmachung des Hinweises auf die Patenterteilung: **05.12.84 Patentblatt 84/49**

(84) Benannte Vertragsstaaten: **AT BE CH DE FR GB IT LI NL SE**

(56) Entgegenhaltungen:
**EP - A - 0 005 153**
**EP - A - 0 010 616**
**DE - A - 2 721 917**

**Chemical Abstracts Band 81, Nr. 5, August 1974, Columbus, Ohio, USA, S. TAKAHASHI et al. "Agricultural pesticides and herbicides containing disulfide derivatives", Seite 154, Spalte 2, Abstract Nr. 22237g**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17E, D-6710 Frankenthal (DE)**
Erfinder: **Ziegler, Hans, Dr., Am Speyerweg 48, D-6704 Mutterstadt (DE)**
Erfinder: **Mappes, Celia Joan, Dr., Wiesenweg 145, D-6721 Westheim (DE)**
Erfinder: **Pommer, Ernst-Heinrich, Dr., Berliner Platz 7, D-6703 Limburgerhof (DE)**

**EP 0 040 344 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues, wertvolles 4-Nitro-2-trichlormethylbenzolsulfensäurederivat mit guter fungizider Wirkung, Verfahren zu seiner Herstellung, Fungizide, die diese Verbindung enthalten, und Verfahren zur Bekämpfung von Pilzen mit dieser Verbindung.

Es ist bekannt, N-Trichlormethylthiotetrahydrophthalimid (Chemical Week 1972, June 21, Seite 46), Tetramethylthiuramdisulfid (Chemical Week 1972, July 26, Seite 39), 2-Thiocyanomethylthio-benzothiazol (Farm Chemicals Handbook 1976, Seite D 43), das Mangansalz der Ethylenbisdithiocarbaminsäure (Chemical Week 1972, July 26, Seite 22) oder das 2,4,5,6-Tetrachlor-isophthalodinitril (Chemical Week 1972, June 21, Seite 55) als Fungizide zu verwenden. Ihre Wirkung ist jedoch nicht befriedigend.

Es ist ferner bekannt, 4-Nitro-2-trichloromethylphenyl-disulfide (DE-OS 2 810 698) und 4-Nitro-2-trichlormethylphenyl-sulfenamide (EP-Anmeldung Nr. 79 103 662) als Fungizide zu verwenden.

Es wurde gefunden, dass Pyridin-1-oxid-2-yl-dithio-(4-nitro-2-trichlormethylbenzol) eine bessere fungizide Wirksamkeit hat als die bekannten Wirkstoffe.

Die Herstellung der neuen Verbindungen erfolgt beispielsweise durch Umsetzung von 4-Nitro-2-trichlormethyl-benzolsulfensäurechlorid mit 2-Mercaptopyridin-N-oxid in einem inerten Lösungsmittel bei Temperaturen von −20 bis 150 °C gegebenenfalls unter Zusatz einer säurebindenden Verbindung.

Für die Umsetzungen kommen als inerte Lösungsmittel beispielsweise Carbonsäuren, wie Essigsäure; gesättigte aliphatische oder cyclische Ether, wie Diethylether, Tetrahydrofuran, Dioxan; aliphatische Carbonsäureester wie Essigsäureethylester, Essigsäurebutylester; niedere Alkohole, wie Isobutanol; chlorierte Kohlenwasserstoffe, wie Dichlormethan; aromatische Kohlenwasserstoffe, wie Benzol, Toluol in Betracht.

Von den genannten Lösungsmitteln sind insbesondere bevorzugt Diethylether, Tetrahydrofuran und Essigsäure.

Als säurebindende Verbindungen können organische und anorganische Basen, wie NaOH, $Na_2CO_3$, Natriumacetat, Kaliumacetat, Triethylamin, Pyridin eingesetzt werden. Natrium- bzw. Kaliumacetat wird bevorzugt.

Die bevorzugte Temperatur liegt bei 0 bis 60 °C für die Umsetzung. Verbindungen, die eine basische Gruppe enthalten, können auch als Hydrochloride hergestellt werden.

Die Ausgangsverbindung 4-Nitro-2-trichlormethylbenzolsulfensäurechlorid kann nach dem in der DE-OS 2 721 917 beschriebenen Verfahren hergestellt werden.

Beispiel 1

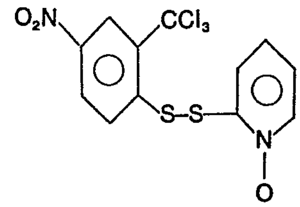

Pyridin-1-oxid-2-yl-dithio-(4-nitro-2-trichlormethylbenzol)

61,4 g 4-Nitro-2-trichlormethylbenzolsulfensäurechlorid werden in 500 ml Eisessig gelöst und bei 20 °C mit 25,4 g 2-Mercaptopyridin-N-oxid und 16,4 g Natriumacetat versetzt. Nach 12 Stunden wird mit Wasser versetzt, abgesaugt und über Kaliumhydroxid getrocknet. Aus Ether erhält man 75 g (93% d. Theorie) Pyridin-1-oxid-2-yl-dithio-(4-nitro-2-trichlormethylbenzol) vom Schmp. 167 °C.

Analyse

ber.:

C 36,2 H 1,8 Cl 26,8 N 7,0 S 16,1

gef.:

C 36,1 H 2,0 Cl 27,0 N 6,9 S 16,2

als Hydrochlorid: Schmp.: 166 °C.

Die neue Verbindung besitzt eine sehr gute fungitoxische Wirksamkeit gegenüber verschiedenen Arten von Schadpilzen, die im Pflanzenschutz oder Materialschutz wirtschaftlich bedeutend sind. Sie ist beispielsweise sehr gut geeignet zur Bekämpfung von Septoria nodorum und Septoria tritici an Weizen, Helminthosphorium-Arten an Getreide, Pythium-Arten an Leguminosen und Baumwolle, Cercospora-Arten an Erdnüssen, Rüben, Kaffee, Mycosphaerella an Bananen, Piricularia an Reis. Die neue Verbindung kann auch zum Materialschutz eingesetzt werden. Folgende materialschädigende Pilze können beispielsweise bekämpft werden: Aspergillus niger, Penicillium glaucum, Chaetomium globosum, Paecilomyces variotii, Cladosporium herbarum, Pullularia pullulans. Darüber hinaus zeichnen sich einige Verbindungen durch eine sehr gute bakterizide Wirksamkeit, beispielsweise gegen Staphylococcus aureus, aus. Bei der Anwendung als Fungizide für den Materialschutz, z.B. als Fungizide für Anstrichfarben, betragen die Aufwandmengen 0,5 bis 5% Wirkstoff, bezogen auf das Gesamtgewicht der zu konservierenden Stoffe. Die fungiziden Mittel enthalten 0,1 bis 95% (Gew.%) Wirkstoff, vorzugsweise 0,5 bis 90%. Die Aufwandmengen liegen je nach Art des gewünschten Effektes zwischen 0,001 und 3 kg oder mehr, vorzugsweise jedoch zwischen 0,01 und 1 kg Wirkstoff/ha. Der neue Wirkstoff kann auch als fungizid wirksamer Bestandteil öliger Holzschutzmittel zum Schutz von Holz gegen holzzerstörende oder holzverfärbende Pilze eingesetzt werden. Die Anwendung erfolgte in der Weise, dass man das Holz mit diesen Mitteln behandelt, beispielsweise tränkt oder anstreicht.

Der Wirkstoff kann als solcher, in Form seiner Formulierungen oder den daraus bereiteten Anwendungsformen z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate z.B. Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und evtl. Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäure, Alkylarylsulfonate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenyl, -Octylphenol, -Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Silikate, Talkum, Kaolin, Kalkstein, Kalk, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden- Holz- und Nussschalenmehl, Cellulosepulver und andere feste Trägerstoffe.

Der Wirkstoff kann auch mit anderen bekannten Fungiziden gemischt werden. In vielen Fällen erhält man dabei eine Vergrösserung des fungiziden Wirkungsspektrums; bei einer Anzahl dieser Fungizidmischungen treten synergistische Effekte auf, d.h. die fungizide Wirksamkeit des Kombinationsproduktes ist grösser als die der addierten Wirksamkeit der Einzelkomponenten.

Die Zumischung dieser Mittel zu den erfindungsgemässen Fungiziden kann im Gewichtsverhältnis 1 : 10 bis 10 : 1 erfolgen.

Sie können gegebenenfalls auch erst unmittelbar vor der Anwendung (Tankmischung) zugesetzt werden.

Beispiel I

10 Gewichtsteile der Verbindung 1 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Äthylenoxid an 1 Mol Ricinusöl besteht. Durch Ausgiessen und feines Verteilen der Lösung in Wasser erhält man eine wässrige Dispersion.

Beispiel II

20 Gewichtsteile der Verbindung 1 werden in einer Mischung gelöst, die aus 640 Gewichtsteilen Cyclohexanon, 30 Gewichtsteilen Isobutanol, 5 Gewichtsteilen des Anlagerungsproduktes von 7 Mol Äthylenoxid an 1 Mol Isooctylphenol und 5 Gewichtsteilen des Anlagerungsproduktes von 40 Mol Äthylenoxid an 1 Mol Ricinusöl besteht. Durch Eingiessen und feines Verteilen der Lösung in Wasser erhält man eine wässrige Dispersion.

Beispiel III

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in Wasser erhält man eine wässrige Dispersion.

Beispiel IV

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 70 Gewichtsteilen pulverförmigem Kieselsäuregel gut vermischt und in einer Hammermühle vermahlen. Durch feines Verteilen der Mischung in Wasser erhält man eine Spritzbrühe.

Beispiel V

3 Gewichtsteile der Verbindung 1 werden mit 97 Gewichtsteilen feinteiligem Kaolin innig vermischt. Man erhält auf diese Weise ein Stäubemittel, das 3 Gew.% des Wirkstoffs enthält.

Beispiel VI

30 Gewichtsteile der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VII

40 Gewichtsteile des Wirkstoffs 1 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit Wasser erhält man eine verdünnte wässrige Dispersion.

Beispiel VIII

20 Teile des Wirkstoffs 1 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Folgende bekannten Vergleichsmittel wurden für die folgenden Versuche verwendet:

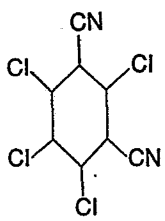

Vergleichsmittel A

$[-S-CS-NH-CH_2-CH_2-NH-CS-S-Mn-]_x$

$x > 1$

Vergleichsmittel B

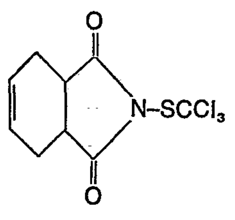

Vergleichsmittel C

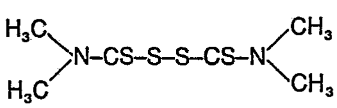

Vergleichsmittel D

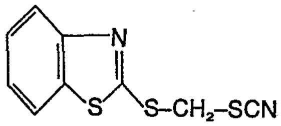

Vergleichsmittel E

Septoria nodorum an Weizen

Blätter von in Töpfen gewachsenen Weizenpflanzen der Sorte «Jubilar» werden mit wässrigen Spritzbrühen, die 0,05 und 0,1% (Gewichtsprozent) Wirkstoff emulgiert enthalten, tropfnass gespritzt. Nach dem Antrocknen des Spritzbelags werden die Pflanzen mit einer Sporenaufschwemmung des Pilzes Septoria nodorum besprüht und bei 16 bis 18°C in eine Kammer mit hoher Luftfeuchtigkeit gestellt, um optimale Bedingungen für die Pilzentwicklung zu erhalten. Nach 14 Tagen hat sich die Krankheit auf den unbehandelten Kontrollpflanzen so stark entwickelt, dass die entstandenen Blattflecken den überwiegenden Teil der Blätter bedecken.

Das Ergebnis des Versuches zeigt, dass der neue Wirkstoff eine bessere fungizide Wirkung hat als das bekannte Vergleichsmittel A.

Fungizide Wirksamkeit gegenüber Aspergillus niger

Der Wirkstoff wird einer für das Wachstum des Pilzes Aspergillus niger optimal geeigneten Nährlösung in Mengen von 100, 50, 25 und 10 Gewichtsteilen pro Million Teile Nährlösung zugesetzt. Es werden jeweils 20 ml der so behandelten Nährlösung in 100 ml Glaskolben mit 0,3 mg Aspergillus-Pilzsporen beimpft. Die Kolben werden 120 Stunden lang bei 36°C erwärmt und anschliessend das Ausmass der Pilzentwicklung, das bevorzugt auf der Nährlösungsoberfläche erfolgt, beurteilt.

Das Ergebnis des Versuches zeigt, dass der neue Wirkstoff 1 eine bessere fungizide Wirkung hat als die bekannten Vergleichsmittel B, C, D und E sowie die in Beispiel 26 der DE-OS 2 810 698 beschriebenen Wirkstoffe 2, 5 und 10 sowie die in Beispiel 51 der EP-Anmeldung Nr. 79 103 662 beschriebenen Wirkstoffe 2, 9 und 12.

**Patentansprüche für die Vertragsstaaten: BE, CH, DE, FR, GB, IT, Li, NL, SE**

1. Pyridin-1-oxid-2-yl-dithio-(4-nitro-2-trichlormethylbenzol).

2. Fungizid, enthaltend Pyridin-1-oxid-2-yl-dithio-(4-nitro-2-trichlormethylbenzol).

3. Fungizid, enthaltend einen festen oder flüssigen Trägerstoff und Pyridin-1-oxid-2-yl-dithio-(4-nitro-2-trichlormethylbenzol).

4. Verfahren zur Herstellung eines Fungizids, dadurch gekennzeichnet, dass man einen festen oder flüssigen Trägerstoff vermischt mit Pyridin-1-oxid-2-yl-dithio-(4-nitro-2-trichlormethylbenzol).

5. Verfahren zur Bekämpfung von Pilzen, dadurch gekennzeichnet, dass man die Pilze oder die vor Pilzbefall zu schützenden Gegenstände behandelt mit Pyridin-1-oxid-2-yl-dithio-(4-nitro-2-trichlormethylbenzol).

6. Verfahren zur Herstellung von Pyridin-1-oxid-2-yl-dithio-(4-nitro-2-trichlormethylbenzol), dadurch gekennzeichnet, dass man 4-Nitro-2-trichlormethylphenylsulfenchlorid mit 2-Mercaptopyridin-N-oxid in einem inerten Lösungsmittel bei −20 bis 150 °C umsetzt.

**Patentansprüche für den Vertragsstaat: AT**

1. Fungizid, enthaltend Pyridin-1-oxid-2-yl-dithio-(4-Nitro-2-trichlormethylbenzol).

2. Fungizid, enthaltend einen festen oder flüssigen Trägerstoff und Pyridin-1-oxid-2-yl-dithio-(4-Nitro-2-trichlormethylbenzol).

3. Verfahren zur Herstellung eines Fungizids, dadurch gekennzeichnet, dass man einen festen oder flüssigen Trägerstoff vermischt mit Pyridin-1-oxid-2-yl-dithio-(4-Nitro-2-trichlormethylbenzol).

4. Verfahren zur Bekämpfung von Pilzen, dadurch gekennzeichnet, dass man die Pilze oder die vor Pilzbefall zu schützenden Gegenstände behandelt mit Pyridin-1-oxid-2-yl-dithio-(4-Nitro-2-trichlormethylbenzol).

5. Verfahren zur Herstellung von Pyridin-1-oxid-2-yl-dithio-(4-Nitro-2-trichlormethylbenzol), dadurch gekennzeichnet, dass man 4-Nitro-2-trichlormethylphenylsulfenchlorid mit 2-Mercaptopyridin-N-oxid in einem inerten Lösungsmittel bei −20 bis 150 °C umsetzt.

**Claims for the Contracting states: BE, CH, DE, FR, GB, IT, Li, NL, SE**

1. N-Oxo-pyridin-2-yl-dithio-(4-nitro-2-trichloromethylbenzene).

2. A fungicide containing N-oxo-pyridin-2-yl-dithio-(4-nitro-2-trichloromethylbenzene).

3. A fungicide containing a solid or liquid carrier and N-oxo-pyridin-2-yl-dithio-(4-nitro-2-trichloromethylbenzene).

4. A process for the production of a fungicide, wherein a solid or liquid carrier is mixed with N-oxo-pyridin-2-yl-dithio-(4-nitro-2-trichloromethylbenzene).

5. A process for combating fungi, wherein the fungi or the objects to be protected against fungus attack are treated with N-oxo-pyridin-2-yl-dithio-(4-nitro-2-trichloromethylbenzene).

6. A process for the preparation of N-oxo-pyridin-2-yl-dithio-(4-nitro-2-trichloromethylbenzene), wherein 4-nitro-2-trichloromethylbenzenesulfenyl chloride is reacted with 2-mercaptopyridine-N-oxide in an inert solvent at from −20 to 150 °C.

**Claims for the Contracting State: AT**

1. A fungicide containing N-oxo-pyridin-2-yl-dithio-(4-nitro-2-trichloromethylbenzene).

2. A fungicide containing a solid or liquid carrier and N-oxo-pyridin-2-yl-dithio-(4-nitro-2-trichloromethylbenzene).

3. A process for the production of a fungicide, wherein a solid or liquid carrier is mixed with N-oxo-pyridin-2-yl-dithio-(4-nitro-2-trichloromethylbenzene).

4. A process for combating fungi, wherein the fungi or the objects to be protected against fungus attack are treated with N-oxo-pyridin-2-yl-dithio-(4-nitro-2-trichloromethylbenzene).

5. A process for the preparation of N-oxo-pyridin-2-yl-dithio-(4-nitro-2-trichloromethylbenzene), wherein 4-nitro-2-trichloromethylbenzenesulfenyl chloride is reacted with 2-mercaptopyridine-N-oxide in an inert solvent at from −20 to 150 °C.

**Revendications pour les Etats contractants: BE, CH, DE, FR, GB, IT, Li, NL, SE**

1. Pyridin-1-oxyd-2-yl-dithio-(4-nitro-2-trichlorométhylbenzène).

2. Fongicide, contenant du pyridin-1-oxyd-2-yl-dithio-(4-nitro-2-trichlorométhylbenzène).

3. Fongicide, contenant un support solide ou liquide et du pyridin-2-oxyd-2-yl-dithio-(4-nitro-2-trichlorométhylbenzène).

4. Procédé de préparation d'un fongicide, caractérisé par le fait que l'on mélange un support solide ou liquide avec du pyridin-1-oxyd-2-yl-dithio-(4-nitro-2-trichlorométhylbenzène).

5. Procédé de lutte contre des champignons, caractérisé par le fait que l'on traite les champignons ou les objets à protéger contre l'attaque des champignons avec du pyridin-1-oxyd-2-yl-dithio-(4-nitro-2-trichlorométhylbenzène).

6. Procédé de préparation de pyridin-1-oxyd-2-yl-dithio-(4-nitro-2-trichlorométhylbenzène, caractérisé par le fait qu'on fait réagir dans un solvant inerte, entre −20 et 150 °C, du chlorure de 4-nitro-2-trichlorométhylphénylsulfène avec du N-oxyde de 2-mercaptopyridine.

**Revendications pour l'Etat contractant: AT**

1. Fongicide, contenant du pyridin-1-oxyd-2-yl-dithio-(4-nitro-2-trichlorométhylbenzène).

2. Fongicide, contenant un support solide ou liquide et du pyridin-1-oxyd-2-yl-dithio-(4-nitro-2-trichlorométhylbenzène).

3. Procédé de préparation d'un fongicide, caractérisé par le fait que l'on mélange un support solide ou liquide avec du pyridin-1-oxyd-2-yl-dithio-(4-nitro-2-trichlorométhylbenzène).

4. Procédé de lutte contre des champignons, caractérisé par le fait que l'on traite les champignons ou les objets à protéger contre l'attaque des champignons avec du pyridin-1-oxyd-2-yl-dithio-(4-nitro-2-trichlorométhylbenzène).

5. Procédé de préparation de pyridin-1-oxyd-2-yl-dithio-(4-nitro-2-trichlorométhylbenzène, caractérisé par le fait qu'on fait réagir dans un solvant inerte, entre −20 et 150 °C, du chlorure de 4-nitro-2-trichlorométhylphénylsulfène avec du N-oxyde de 2-mercaptopyridine.